# FASCICULE DE BREVET EUROPEEN

(11) **EP 3 579 929 B1**
(45) Date de publication et mention de la délivrance du brevet: **13.04.2022**
(21) Numéro de dépôt: 18704205.6
(22) Date de dépôt: 06.02.2018
(51) Int. Cl.: A61Q 19/00, A61Q 19/08, A61Q 19/10, A61K 8/97, A61K 36/27

(54) **EXTRAITS DE LA LIANEMARSDENIA CUNDURANGO**
MARSDENIA CUNDURANGO
MARSDENIA CUNDURANGO

(30) Priorité: 09.02.2017 FR 1770125
(43) Date de publication de la demande: 18.12.2019
(73) Titulaire: ISP Investments LLC, Wilmington, DE 19805 (US); Jafer Enterprises R&D, S.L. (Sociedad Unipersonal), 08403 Granollers (Barcelona) (ES)
(72) Inventeur: CLAY, Anne, 08960 Sant Just Desvern, Barcelona (ES); DOMLOGE, Nouha, 06650 Opio (FR); IMBERT, Isabelle, 06400 Cannes (FR); CUCUMEL, Karine, 06650 Opio (FR); CICCHETTI, Esmeralda, 40260 Castets (FR); MUR, Ludivine, 06580 Pegomas (FR); GARNIER, Sébastien, 06650 Le Rouret (FR); DUROURE, Leslie, 06370 Mouans Sartoux (FR)
(74) Mandataire: Miquel, Corinne
(86) Numéro de dépôt international: PCT/EP2018/052887
(87) Numéro de publication internationale: WO 2018/146066

(56) Documents cités:
- CN-A- 105 535 515
- JP-B2- 3 754 025

## Description

L'invention concerne un extrait d'écorce et/ou de bois de la liane de *Marsdenia cundurango,* son procédé de préparation, des compositions cosmétiques le comprenant et leurs utilisations cosmétiques.

Dans le cadre de la présente invention, la plante concernée est une liane grimpante dont le nom botanique est *Marsdenia cundurango* Rchb. F. et qui appartient à la famille des *Apocynaceae.* Cette plante pousse entre 2000 et 3000 mètres d'altitude. Le condurango est originaire d'Amérique du Sud: il pousse sur le versant ouest de la Cordillère des Andes (en Equateur, au Pérou et en Colombie). Le condurango est une liane aux branches velues. Le tronc atteint un diamètre de 10 cm. Les jeunes pousses grimpantes sont recouvertes d'un fin duvet feutré; les anciennes branches et le tronc sont enveloppés d'une écorce grise. Le latex produit par la plante est vénéneux à l'état frais. Les feuilles du condurango, allongées en forme de cœur, sont coriaces et pubescentes. Les petites fleurs de couleur blanche verdâtre poussent en inflorescence ombelliforme à l'aisselle des feuilles. L'écorce de condurango utilisée comme substance active provient d'arbustes cultivés. La substance active appelée « écorce de condurango » (en latin: condurango cortex) est en fait l'écorce séchée des branches et du tronc de la plante. Transformée en poudre fine, l'écorce de condurango est utilisée directement comme médicament. Coupée finement ou broyée grossièrement, elle sert de base pour préparer des infusions, des extraits (par ex. Condurango extractum fluidum) ou un vin (condurango vinum). Ce remède est amer et légèrement irritant. Il est composé notamment de la substance amère condurangine (un mélange de plusieurs glucocides), de petites quantités de flavonoïdes et de coumarine.

La partie de plante utilisée dans la présente invention est l'écorce et/ou le bois (branche et tronc) de la liane.

Il est connu des revues bibliographiques, un certain nombre de composés issus de l'écorce de *Marsdenia cundurango* (ci-après dénommé « Condurango »). Une grande partie des études d'isolement faite sur cette matière a permis de mettre en évidence des composés de type condurangosides ou condurangoglycosides (Berger, S. et al., Structural Revision of Pregnane Ester Glycosides from Condurango Cortex and New Compounds. Phytochemistry 1988, 27 (5), 1451-1458; Umehara, K. et al., Studies on Differentiation Inducers. IV. Pregnane Derivatives from Condurango Cortex. Chem. Pharm. Bull. (Tokyo) 1994, 42, 611-616; Tschesche, R. et al. Digitanol Glycosides. XVI. Structure of Kondurangogenins A and C2.Tetrahedron 1967, 23, 1461-1471).

Cependant, l'écorce de Condurango présente une diversité chimique notable car d'autres familles de métabolites secondaires sont aussi décrites dans la littérature. La présence de certains flavonoides comme la rutine, la quercitrine, l'hyperine, la trifoline ; de quelques phénylpropanoides, comme les acides chlorogénique, néochlorogénique et cafféique ; ou encore de vanilline et d'un dérivé de saponarine a été rapportée dans les écorces (Koch, H. et al. Components of Condurango Bark. 2. Pharm. Acta Helv. 1982, 57, 211-214. Les condurangamines A et B ont aussi été isolées et décrites suite à une étude sur cette matière (Kindl, H. et al., Biosynthesis of Cyclitols. XIII. Occurrence and Biosynthesis of Cyclitols in Asclepiadaceae. Phytochem. Elsevier 1966, 5, 1091-1102).

Il est par ailleurs connu d'un rapport technique sur *Marsdenia cundurango* rédigé par le Dr. Taylor en 2006 (Taylor, L. Technical Data Report for Condurango (Marsdenia Cundurango). 2006) que la plante comprend de nombreux composés. Parmi les nombreux composés cités connus dans *Marsdenia cundurango,* ce rapport technique cite notamment le cinnamate de β-amyrine présent dans l'écorce et le bois. Le rapport indique clairement que la plante était tout d'abord utilisée par les populations locales pour résoudre des problèmes d'ordre digestifs et qu'elle a d'ailleurs été incluse dans certaines pharmacopées.

Ainsi, aujourd'hui, parmi les activités biologiques connues de Condurango, on peut citer également ses propriétés anti-oxydantes et anti-inflammatoires (de las Heras, B. Et al., Antiinflammatory and Antioxidant Activity of Plants Used in Traditional Medicine in Ecuador. J. Ethnopharmacol. 1998, 61 (2), 161-166).

Plus récemment, le potentiel anti-cancéreux de Condurango a été mis en évidence par plusieurs équipes. Sikdar *et al.* se sont ensuite attachés à démontrer l'activité d'un extrait global de condurango, puis d'un ester glycosylé de la condurangogenine A contre le cancer du poumon (Sikdar, S. et al. , Anti-Lung Cancer Potential of Pure Esteric-Glycoside Condurangogenin A against Nonsmall-Cell Lung Cancer Cells in Vitro via p21/p53 Mediated Cell Cycle Modulation and DNA Damage-Induced Apoptosis. Pharmacogn. Mag. 2015, 11, 73-85; Sikdar, S. et al. Condurango Glycoside-Rich Components Stimulate DNA Damage-Induced Cell Cycle Arrest and ROS-Mediated Caspase-3 Dépendent Apoptosis through Inhibition of Cell-Proliferation in Lung Cancer, in Vitro and in Vivo. Environ. Toxicol. Pharmacol. 2014, 37, 300-314; Sikdar, S. et al., Ethanolic Extract of Marsdenia Condurango Ameliorates Benzo[a]pyrene-Induced Lung Cancer of Rats: Condurango Ameliorates BaP-Induced Lung Cancer in Rats. J. Pharmacopuncture 2014, 17, 7-17).

De leurs côtés, Bishayee *et al.* ont aussi montré que le condurango glycoside A peut avoir un potentiel anti-cancéreux (Bishayee, K. et al., Condurango-Glycoside-A Fraction of Gonolobus Condurango Induces DNA Damage Associated Senescence and Apoptosis via ROS-Dependent p53 Signalling Pathway in HeLa Cells. Mol. Cell. Biochem. 2013, 382, 173-183).

Enfin, des études épigénétiques ont été menées très récemment (Saha et al, , Ultra-Highly Diluted Plant Extracts of Hydrastis Canadensis and Marsdenia Condurango Induce Epigenetic Modifications and Alter Gene Expression Profiles in HeLa Cells in Vitro. J. Integr. Med. 2015, 13 , 400-411).

Les Demandeurs ont maintenant développé un procédé d'extraction nouveau appliqué à l'écorce et/ou le bois de la liane de Condurango séchée permettant d'obtenir un extrait riche en composés cinnamates de terpényles qui présente un ensemble d'activités cosmétiques non encore décrites.

Ainsi l'extrait de Condurango selon la présente invention s'est avéré utile pour améliorer la fonction barrière de la peau, pour améliorer la protection de la peau vis-à-vis des toxines, pour améliorer la détoxination de la peau et pour diminuer les signes du vieillissement. Les demandeurs ont également mis en évidence le pouvoir de l'extrait d'écorce de la liane de *Marsdenia cundurango,* d'augmenter l'expression de récepteurs du goût amer (TAS2R) dans les cellules de la peau. Cette famille des récepteurs, et en particulier le récepteur TAS2R38 qui a été identifié dans les cellules cutanées, est activé par différents métabolites de plantes et certaines substances chimiques de synthèse. L'activation de ce récepteur est liée à l'amélioration de la fonction de barrière cutanée (Wölfle U et al. Expression and functional activity of the bitter taste receptors TAS2R1 and TAS2R38 in human keratinocytes. Skin Pharmacol Physiol. 2015; 28(3):137-46).

Dans le domaine cosmétique, il est connu le document faisant référence au genre *Marsdenia* dans son ensemble, c'est le document JP3754025 de Noevir KK, 8 mars 2006. Ce document décrit l'utilisation d'un extrait de plante du genre *Marsdenia tenacissima* comme agent antioxydant pour prévenir le vieillissement de la peau. Il est précisé que toute la plante doit être utilisée et les exemples confirment une extraction à partir de la plante entière, notamment les racines, les feuilles, la tige, le bois et les fleurs. Le procédé d'extraction utilise de l'eau ou des alcools et peut également utiliser un fluide supercritique, dans ce cas sans ajout de co-solvant, et ce en appliquant une pression de 15MPa et une température de 40°C.

Le procédé d'extraction selon la présente invention, mis au point par les Demandeurs sur spécifiquement l'écorce et/ou le bois de la liane de Condurango, est nouveau par rapport aux documents cités ci-dessus. Ce procédé est optimisé pour sélectionner et garantir dans l'extrait obtenu une teneur élevée en molécules cibles d'intérêt, à savoir les composés cinnamates de terpényles.

Les composés d'intérêt sont selon l'invention sont notamment les composés cinnamates de terpényles suivants:
- le cinnamate de β-amyrine, de formule suivante : 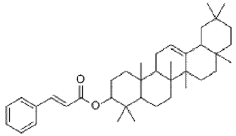
- le cinnamate de 24-méthylènecycloartanyle, 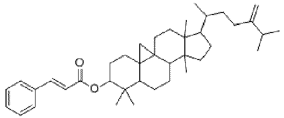
- le cinnamate de butyrospermyle, de formule suivante : 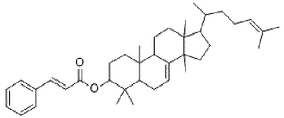
- le cinnamate de cycloartényle, de formule suivante : 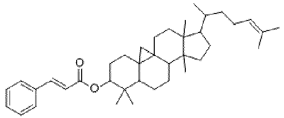 et,
- le cinnamate de cyclofontumiényle, de formule suivante :

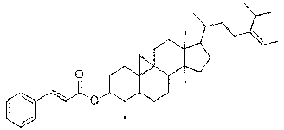

Le cinnamate de 24-méthylènecycloartanyle et le cinnamate de cycloartényle sont référencés dans les travaux de Lai *et al.* sur le fruit de *Gymnema alternifolium* en précisant une activité antimicrobienne (Lai, J. S. et al., Studies on the Constituents of the Fruits of Gymnema Alternifolium (Lour.) Merr. Proc. Natl. Sci. Counc. Repub. China Part Phys. Sci. Eng. 1987, 11, 203-208).

Pour le composé cinnamate de butyrospermyle, la publication d'Akihisa *et al.* discute du potentiel anti-inflammatoire et anti-tumoral des acétates et des cinnamates de triterpènes du beurre de karité (Akihisa, T. et al., Anti-Inflammatory and Chemopreventive Effects of Triterpene Cinnamates and Acetates from Shea Fat. J. Oleo Sci. 2010, 59, 273-280).

On connaît également d'un abrégé datant de 1942 que le cinnamate de β-amyrine est présent dans l'écorce de Condurango (Kern, W.; Haselbeck, W. Components of Condurango Bark. IV. Arch. Pharm. Ber. Dtsch. Pharm. Ges. 1942, 280, 277-292) mais sans indiquer aucune précision sur son potentiel biologique. Le potentiel biologique digestif est précisé dans le rapport technique sur *Marsdenia cundurango* cité ci-avant et rédigé par le Dr. Taylor en 2006 (Taylor, L. Technical Data Report for Condurango (Marsdenia Cundurango).

Néanmoins, il n'était ni connu ni suggéré que l'écorce et/ou le bois de Condurango puisse comprendre une teneur élevée en molécules cibles d'intérêt selon l'invention, à savoir les différents cinnamates de terpényles. Il n'était pas non plus connu ni suggéré que le procédé d'extraction spécifique mis en œuvre selon la présente invention puisse permettre de sélectionner et concentrer à partir de l'écorce et/ou le bois de Condurango les composés d'intérêt selon l'invention.

L'invention a donc pour premier objet un procédé d'obtention d'un extrait de *Marsdenia cundurango* comprenant des composés cinnamates de terpényles, caractérisé en ce qu'il comprend les étapes suivantes :
a) on recueille l'écorce et/ou le bois des lianes de *Marsdenia cundurango* qui sont séchés puis broyés;
b) on réalise une extraction avec un dioxyde de carbone (CO₂) à l'état supercritique, à une température comprise entre 45 et 55°C et à une pression comprise entre 200 et 260 bar au sein de l'extracteur;
c) on récupère l'extrait de *Marsdenia cundurango,*
d) éventuellement on purifie l'extrait obtenu à l'étape c).

L'invention a pour second objet un extrait provenant de l'écorce et/ou du bois de la liane de *Marsdenia cundurango* contenant des composés cinnamates de terpényles, obtenu par le procédé ci-dessus.

L'invention a pour troisième objet une composition cosmétique comprenant, à titre d'agent actif, une quantité efficace d'un extrait de *Marsdenia cundurango,* obtenu par le procédé ci-dessus et un excipient physiologiquement acceptable.

L'invention a pour quatrième objet l'utilisation cosmétique d'une composition pour améliorer la fonction barrière de la peau, pour améliorer la protection vis-à-vis des toxines, pour augmenter les capacités de détoxination de la peau et pour diminuer les signes du vieillissement.

Enfin, l'invention a pour cinquième objet une méthode de traitement cosmétique pour augmenter l'expression des récepteurs du goût amer dans les cellules de la peau, comprenant l'application topique d'une composition de l'invention.

Dans cette description, à moins qu'il n'en soit spécifié autrement dans le texte, il est entendu que:
- lorsqu'un intervalle est donné, il inclut les bornes supérieure et inférieure dudit intervalle, ainsi que toutes les valeurs numériques comprises entre les bornes de l'intervalle.
- les % sont exprimés en poids/poids ou encore % massique dans le texte, sauf indication contraire.

Dans la présente invention, on entend par :
✔ « quantité efficace » la quantité nécessaire d'extrait de condurango pour obtenir le résultat recherché, à savoir, permettre notamment d'obtenir une protection cutanée vis à vis des toxines, ou une augmentation de l'expression du marqueur TAS2R, sans que cette quantité ne soit toxique.
✔ « Extrait de condurango », « extrait de la ou des lianes de *Marsdenia cundurango »* ou « extrait de *Marsdenia cundurango »* peuvent être utilisés indifféremment et désignent un extrait provenant de l'écorce et/ou du bois de la plante *Marsdenia cundurango* obtenu selon le procédé de l'invention.
✔ « écorce et/ou bois » l'écorce entourant les branches et le tronc de la plante et/ou le bois (branches et tronc de la plante).
✔ « molécules d'intérêt » les composés cinnamates de terpényles et molécules apolaires extraites par le procédé selon l'invention.
✔ Co-solvant apolaire : substance non polaire ayant un rôle de solvant en association avec une autre substance
✔ Fluide à l'état supercritique : état physique d'un corps pur chauffé au-delà de sa température critique et compressé au-dessus de sa pression critique. (exemples : dioxyde de carbone ou argon).
✔ « cinnamate de terpényle » les composés de la famille des triterpènes ou des stéroides estérifés en position 3 par l'acide cinnamique.
✔ « application topique », le fait d'appliquer ou d'étaler l'agent actif selon l'invention, ou une composition le contenant, à la surface de la peau, d'une muqueuse ou des phanères.
✔ « toxine », non seulement les substances toxiques produites par les organismes vivants tels que les bactéries, champignons ou animaux venimeux, mais aussi les polluants chimiques et environnementaux qui peuvent entrer en contact avec la peau et causer des dommages.
✔ « polluants chimiques et environnementaux » les polluants présents dans l'environnement, qui sont néfastes pour la peau. Ils peuvent être présents à la fois à l'extérieur, par exemple les particules de moteur diesel, l'ozone, ou les métaux lourds, et/ou à l'intérieur des maisons où la pollution peut notamment être due à la fumée de cigarette ou aux solvants libérés par les peintures, les colles ou les papiers peints, comme le toluène, styrène, xylène, benzaldéhyde.
✔ « détoxination » que l'extrait de condurango améliore le pouvoir de la peau de détecter et d'éliminer les déchets pour un meilleur fonctionnement.
✔ « cosmétiquement acceptable », que les composés d'intérêt ou l'agent actif selon l'invention, ou une composition le contenant, est approprié pour entrer en contact avec la peau ou une muqueuse sans provoquer de réactions de toxicité ou d'intolérance.
✔ « physiologiquement acceptable » qui convient à une utilisation topique, en contact avec la peau humaine, ou à une utilisation par d'autres voies d'administration, par exemple la voie orale ou l'injection dans la peau, sans risque de toxicité, d'incompatibilité, d'instabilité, de réponse allergique.
✔ « signes du vieillissement cutané » toute modification de l'aspect extérieur de la peau due au vieillissement chronologique comme, par exemple, les rides et ridules, les crevasses, les poches sous les yeux, les cernes, le flétrissement, la perte d'élasticité et/ou de tonus de la peau, le ternissement ou le manque d'éclat, les défauts pigmentaires mais également toute modification interne de la peau qui ne se traduit pas systématiquement par un aspect extérieur modifié comme, par exemple, l'amincissement et la perte de densité du derme, l'épaississement de la couche cornée. La peau peut aussi s'épaissir et devenir rugueuse. Les signes du vieillissement incluent également toute dégradation de la peau consécutive à une exposition aux rayonnements UV comme l'apparition prématurée de rides fines autour des yeux et de la bouche et de rides d'expression sur le front; la télangiectasie (petits vaisseaux sanguins dilatés) sur le nez, les joues et le cou, les taches pigmentaires diverses telles que les taches de rousseur et les lentigines solaires, les irrégularités du teint, la perte généralisée de tonus de la peau, la perte de couleur et l'amincissement des lèvres. On entend également toute modification interne de la peau qui ne se traduit pas systématiquement par un aspect extérieur modifié comme, par exemple, l'épaississement des parois des vaisseaux, la modification de la forme des fibroblastes, le ralentissement de la synthèse du collagène et une désorganisation des fibrilles de collagène, une accumulation de matériel anormal et amorphe contenant de l'élastine (l'élastose solaire).
Les signes du vieillissement cutanés se manifestent également au niveau moléculaire et cellulaire, par exemple par le ralentissement de l'autophagie, une voie de dégradation lysosomale, permettant l'élimination des toxines et des composants intercellulaires. L'autophagie médiée par chaperone (CMA) est l'une des formes sélectives d'autophagie, caractérisée par la présence de LAMP2A. Le niveau de LAMP2A à la membrane lysosomale diminue avec l'âge, induisant un défaut de l'activité CMA (E. Bejarano et A.M. Cuervo, Chaperone-mediated Autophagy, Proc Am Thorac Soc, Vol 7, 29-39, 2010 et S. Kaushik, U. Bandyopadhyay, S. Sridhar, R. Kiffin, M. Martinez-Vicente, M. Kon, S. J. Orenstein, E. Wong, A. M. Cuervo, Chaperone-mediated autophagy at a glance, Journal of Cell Science 124, 495-499, 2011).

L'invention a donc pour premier objet un procédé d'obtention d'un extrait de *Marsdenia cundurango* contenant des composés cinnamates de terpényles.

Pour obtenir cet extrait apolaire de *Marsdenia cundurango,* contenant des composés cinnamates de terpényles, on utilise un procédé d'extraction par fluide supercritique:
Un fluide est dit supercritique lorsqu'il est placé dans des conditions de température et de pression au-delà de son point critique (*T_{c}*, *P_{c}*)*.* Les propriétés physiques d'un fluide supercritique (densité, viscosité, diffusion, diffusivité) sont intermédiaires entre celles des liquides et celles des gaz, mais leurs propriétés de dissolution sont considérablement accrues. Il est bien connu d'utiliser du dioxyde de carbone (CO₂) à l'état supercritique car il présente l'avantage d'être un solvant totalement neutre, non toxique, non inflammable, pouvant être mis en œuvre à une température assez basse (31°C) pour une pression supérieure à sa pression critique de 73,8 bar. Cette technique permet de travailler à une température modérée (à partir de 31°C), ce qui ne dénature pas les qualités organoleptiques et les principes actifs de l'extrait obtenu. De plus, après évaporation du CO2 revenu à l'état gazeux, elle permet d'obtenir des extraits exempts de tous résidus du solvant d'extraction. Il est aussi possible d'utiliser d'autres fluides dont notamment l'argon.

Le procédé préféré selon l'invention consiste à utiliser l'extraction par du CO₂ à l'état supercritique.

Ainsi, un premier objet selon l'invention est le procédé d'extraction d'un extrait de *Marsdenia cundurango* comprenant des composés cinnamates de terpényles, lequel procédé comprend les étapes suivantes:
a) on recueille l'écorce et/ou le bois des lianes de *Marsdenia cundurango* qui sont séchés puis broyés;
b) on réalise une extraction avec du dioxyde de carbone (CO₂) à l'état supercritique;
c) on récupère l'extrait de *Marsdenia cundurango,*
d) éventuellement on purifie l'extrait obtenu à l'étape c).

L'extrait obtenu à l'étape c) est pâteux.

L'étape d) de purification de l'extrait obtenu à l'étape c) peut être effectuée par toute technique connue de l'homme du métier et notamment par chromatographie ou par distillation moléculaire.
- La température d'extraction est comprise entre 45 et 55°C.
- La pression au sein de l'extracteur est comprise entre 200 et 260 bar.
- Le rapport massique de solvant (dioxyde de carbone) par rapport à la quantité de matière première mise en jeu est compris entre 10 et 70, avantageusement entre 25 et 55 et préférentiellement entre 35 et 45.

Dans un mode de réalisation plus préférentiel du procédé selon l'invention, l'extraction à l'étape b) est effectuée à une température d'environ 50°C et une pression d'environ 230 bar au sein de l'extracteur pour obtenir un extrait apolaire à l'étape c).

Dans encore un autre mode de réalisation préférentiel, l'extrait de *Marsdenia cundurango* obtenu du procédé selon l'invention contient au moins 10 % en poids de composés cinnamates de terpényles

Dans un mode de réalisation très préférentiel selon l'invention, on utilise en outre au moins un co-solvant apolaire à l'étape b).

Les co-solvants apolaires utilisables sont de préférence des solvants agro-sourcés tels que par exemple des huiles végétales, des esters d'acides et d'alcools à chaîne plus ou moins longue tels que par exemple l'acétate d'éthyle, le lactate d'éthyle, le propionate d'éthyle, le palmitate d'isopropyle, l'oléate d'éthyle, le stéarate de méthyle, l'oléate d'oleyle, le triéthyl citrate, le triglycéride caprylate/caprate de glycérol ou encore des hydrocarbures agro-sourcés comprenant entre 10 et 14 carbone (C10 à C14), ou une combinaison de ces solvants.

De préférence encore le co-solvant est le palmitate d'isopropyle ou le triglycéride caprylate/caprate de glycérol, de préférence le triglycéride caprylate/caprate de glycérol.

Dans ce mode de réalisation avantageux selon l'invention, l'action du fluide à l'état supercritique dans l'étape b) du procédé est renforcé par le co-solvant, tel qu'un ester gras comme le triglycéride caprylate/caprate de glycérol ou le palmitate d'isopropyle, de préférence encore le triglycéride caprylate/caprate de glycérol, servant de solvant et de support liquide cosmétiquement acceptable aux composés d'intérêt. De préférence le solvant est un solvant agro-sourcé. Par agro-sourcé, on entend des molécules provenant totalement ou partiellement de la biomasse, en effet ces solvants sont composés de carbone renouvelable.

Ainsi, le procédé est avantageusement réalisé selon des critères d'éco-extraction en utilisant un co-solvant apolaire agro-sourcé afin d'obtenir les composés d'intérêt d'une manière facilitée, c'est à dire sans besoin d'étape de purification ultérieure pour éliminer les solvants d'extraction. En effet, le fluide supercritique s'évapore spontanément et le co-solvant sert de support liquide directement utilisable en cosmétique.

Ainsi de manière très avantageuse, le procédé comprend les étapes de :
a. Cryobroyage des écorces et/ou du bois préalablement séchés, puis
b. Extraction par le dioxyde de carbone à l'état supercritique et en présence d'un co-solvant comme le triglycéride caprylate/caprate de glycérol, puis
c. Récupération de l'extrait sous forme liquide et éventuellement
d. Purification de l'extrait.

L'invention a pour second objet un extrait de l'écorce et/ou du bois de la liane de *Marsdenia cundurango* obtenu par le procédé selon l'invention.

Dans un mode de réalisation, l'extrait de l'écorce et/ou du bois de la liane de *Marsdenia cundurango* obtenu par le procédé est pâteux et comprend des composés cinnamates de terpényles en une concentration d'au moins 10% en poids du poids total de l'extrait.

Dans les conditions d'utilisation du dioxyde de carbone à l'état supercritique seul, le procédé selon l'invention permet d'obtenir un extrait pâteux. Il contient comme indiqué dans l'exemple 2 une teneur en molécules d'intérêt (composés cinnamates de terpényles ) d'au moins 10% en poids du poids total de l'extrait. L'extrait peut également être séché et purifié pour obtenir une teneur en molécules d'intérêt allant jusqu'à 100% en poids du poids total de l'extrait. Cet extrait peut ensuite être dilué, dans les conditions indiquées dans le mode préférentiel selon l'invention, pour une utilisation cosmétique.

Dans un autre mode de réalisation préférentiel selon l'invention, lorsque l'extraction est réalisée à l'aide d'un fluide à l'état supercritique et d'au moins un co-solvant, l'extrait de l'écorce et/ou du bois de la liane de *Marsdenia cundurango* est sous forme liquide et comprend des composés cinnamates de terpényles en une concentration de 0,5% à 2% en poids du poids total de l'extrait.

Dans un mode de réalisation plus préférentiel, les composés cinnamates de terpényles de l'extrait sont de formule générale suivante I : 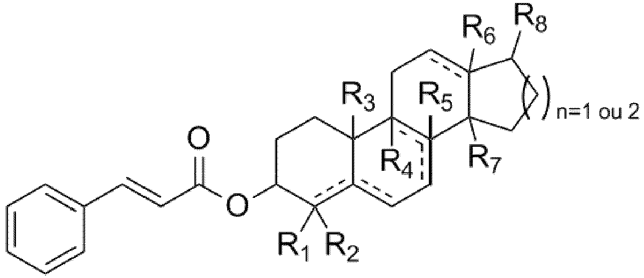 dans laquelle :
- les radicaux R1, R2, R3, R4, R5, R6 et R7 représentent des protons, des méthyles ou des chaînes alkyles oxydées ou non oxydées, saturées ou non saturées.
- le radical R8 représente un groupement alkyle cyclique ou non cyclique, saturé ou non saturé, oxydé ou non oxydé.
- Les pointillés illustrent la présence potentielle d'insaturation à cette position.

Dans un mode de réalisation encore plus préférentiel selon l'invention, les composés cinnamates de terpényles sont respectivement le cinnamate de β-amyrine, le cinnamate de 24-méthylènecycloartanyle, le cinnamate de butyrospermyle, le cinnamate de cycloartényle et le cinnamate de cyclofontumiényle.

La proportion des composés cinnamates de terpényles, selon l'origine de la liane *Marsdenia cundurango* utilisée, obtenue de l'écorce et/ou du bois, est la suivante:
19,4 à 35,7 % de cinnamate de butyrospermyle,
13,2 à 24,6 % de cinnamate de cycloartényle,
16,9 à 31,5 % de cinnamate de β-amyrine,
25,6 à 25,6 % de cinnamate de 24-méthylènecycloartanyle,
2,8 à 5,2 % de cinnamate de cyclofontumiényle.

L'extrait de Condurango selon l'invention est obtenu de préférence à partir de l'écorce de la liane de *Marsdenia cundurango* comme matière de départ. L'extrait obtenu est de type apolaire.

L'écorce de la liane de *Marsdenia cundurango,* récoltée et séchée, provient de préférence d'Amérique du Sud. Elle peut provenir notamment de l'Equateur, du Pérou ou de Colombie.

Lorsque l'extrait obtenu par le procédé selon l'invention est liquide, il comprend avantageusement des composés cinnamates de terpényles en une concentration de 0,5% à 2% en poids du poids total de l'extrait et comprend les composés cinnamates de terpényles, les autres composés apolaires sous forme de trace et le co-solvant d'extraction. Cette concentration en composés d'intérêt peut également être concentrée en milieu liquide après purification par microfiltration, ultrafiltration et/ou nanofiltration pour concentrer l'extrait en composés cinnamates de terpényles par rapport aux autres composés également extraits.

Dans un mode de réalisation préférentiel, l'extrait liquide selon l'invention provient de l'écorce de liane de *Marsdenia cundurango.*

L'invention concerne également l'extrait provenant de l'écorce et/ou de bois de liane de *Marsdenia cundurango* comprenant des composés cinnamates de terpényles et susceptible d'être obtenu par le procédé selon l'invention.

Un troisième objet selon l'invention concerne une composition cosmétique comprenant, à titre d'agent actif, une quantité efficace d'un extrait de *Marsdenia cundurango* et un excipient physiologiquement acceptable.

Dans un mode de réalisation préférentiel selon l'invention, l'extrait est obtenu à partir uniquement de l'écorce de la liane de *Marsdenia cundurango.*

Dans un mode de réalisation préférentiel selon l'invention, l'extrait de *Marsdenia cundurango* est présent dans la composition à une concentration de 0,001 à 10% en poids par rapport au poids total de la composition, préférentiellement de 0,01 et 5,0 % en poids par rapport au poids total de la composition et encore plus préférentiellement de 0,01 et 2,0 % en poids par rapport au poids total de la composition.

Les compositions selon l'invention sont destinées plus particulièrement à une administration par voie topique. Ces compositions doivent donc contenir un milieu cosmétiquement acceptable, c'est-à-dire compatible avec la peau et les phanères, et couvrent toutes les formes galéniques cosmétiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, anti-oxydants, colorants, conservateurs, parfums. Elles peuvent être utilisées comme produit de soin et/ou comme produit de maquillage de la peau.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook ("International Nomenclature of Cosmetic Ingrédients 13ème Ed. 2010) publié par "the Personal Care Products Council, Inc.", Washington, D.C.) décrit une grande variété, sans limitation, d'ingrédients cosmétiques habituellement utilisés dans l'industrie du soin de la peau, qui conviennent pour être utilisés comme ingrédients additionnels dans les compositions selon la présente invention.

Des exemples non limitatifs de ces classes d'ingrédients additionnels incluent : les agents cicatrisants, les agents anti-âge, les agents anti-rides, les agents anti-atrophie, les agents hydratants, les agents adoucissants, , les agents kératolytiques, les agents anti-radicaux libres, les agents anti-séborrhéiques, les agents anti-pelliculaires, les agents modulant la différenciation, la prolifération ou la pigmentation cutanée, les agents accélérateurs de pénétration, les agents desquamants, les agents stimulant ou inhibant la synthèse de mélanine, les agents blanchissants, dépigmentants, ou éclaircissants, les agents propigmentants, les agents auto bronzants, les agents inhibant la NO-synthase, les agents antioxydants, les agents piégeurs de radicaux libres et/ou anti-pollution atmosphérique, les agents anti-glycation, les agents raffermissants, les agents stimulant la synthèse des macromolécules dermiques ou épidermiques et/ou les agents capables de prévenir ou inhiber leur dégradation, les agents stimulant la synthèse de collagène, les agents stimulant la synthèse d'élastine, les agents stimulant la synthèse de décorine, les agents stimulant la synthèse de laminine, les agents stimulant la synthèse de défensine, les agents stimulant la synthèse de chaperon, les agents stimulant la synthèse de d'aquaporine, les agents stimulant la synthèse d'acide hyaluronique, les agents stimulant la synthèse de lipides et de composants du stratum comeum (céramides, acides gras, ...), les agents inhibant la dégradation du collagène, les agents inhibant la dégradation de l'élastine, les agents stimulant la prolifération des fibroblastes, les agents stimulant la prolifération des kératinocytes, les agents stimulant la prolifération des adipocytes, les agents stimulant la prolifération des mélanocytes, les agents stimulant la différenciation des kératinocytes, les agents stimulant la différenciation des adipocytes, les agents inhibant l'acétylcholinestérase, les agents stimulant la synthèse des glycosaminoglycanes, les agents réparant l'ADN, les agents protégeant l'ADN, les agents anti-démangeaison, les agents pour le traitement et/ou le soin de la peau sensible, les agents raffermissants, les agents anti-vergetures, les agents astringents, les agents régulant la production du sébum, les agents dermo-relaxants, , les agents stimulant la réépithélialisation, les facteurs de croissance de cytokine, , les agents agissant sur la circulation et/ ou microcirculation capillaire, les agents inhibant la perméabilité vasculaire, les agents agissant sur le métabolisme cellulaire, les agents destinés à améliorer la jonction dermo-épidermique, les agents induisant la pousse des cheveux et/ou des poils, les agents inhibant ou ralentissant la pousse des cheveux et/ou des poils, les agents myorelaxants, les agents anti-pollution et/ou anti-radicalaires, les agents stimulant la lipolyse, les agents amincissants, les agents anti-cellulite, les agents agissant sur le métabolisme des cellules, les agents nettoyants, les agents de coiffage du cheveu, les stimulants de la pousse des cheveux, les écrans solaires, les écrans totaux, les agents de maquillage, les détergents, les agents émulsifiants, les émollients, les solvants organiques, les agents antiseptiques, les actifs déodorants, les milieux physiologiquement acceptables, les surfactants, les agents abrasifs, les absorbants, les composants esthétiques comme les parfums, les pigments, les teintures, colorants et colorants naturels, les huiles essentielles, les agents de toucher, les astringents cosmétiques, les agents anti-mousse, les antioxydants, les ligants, les additifs biologiques, les enzymes, les inhibiteurs enzymatiques, les inducteurs enzymatiques, les coenzymes, les agents chélatants, les extraits végétaux et dérivés de plantes, les huiles essentielles, les extraits marins, les agents venant d'un procédé de biofermentation et/ou de biotechnologie, les sels minéraux, les extraits cellulaires, les écrans solaires (les agents photoprotecteurs organiques ou minéraux qui sont actifs contre les rayons ultraviolets A et/ou B) les céramides, les peptides, les tampons, les agents de volume, les agents chélatants, les additifs chimiques, les colorants, les biocides cosmétiques, les dénaturants, les agents filmogènes, comme les polymères, pour exacerber les propriétés filmogènes et la substantivité de la composition, les dérivés quaternaires, les agents augmentant la substantivité, les agents opacifiants, les ajusteurs et régulateurs de pH (ex. triethanolamine), les propellants, les agents réducteurs, les séquestrants, les agents décolorants et/ou éclaircissants de la peau, les agents conditionnant de la peau (i.e., humectants, incluant miscellanées et occlusifs), les substances retenant l'humidité, les alpha hydroxy acides, les béta hydroxy acides, les hydratants, les enzymes hydrolytiques épidermiques, les agents apaisants et/ou cicatrisants, les agents traitant la peau, les agents anti-rides, les agents capables de réduire ou traiter les poches sous les yeux, les agents exfoliants, les épaississants, les adoucissants, les polymères gélifiants, les vitamines et leurs dérivés, les agents mouillants, les agents pelants, les agents calmants, les lignanes, les conservateurs (agents antifongiques, agents fongistatiques, agents bactéricides, agents bactériostatiques, i.e.phenoxyethanol et parabens), les anti-UV, agents modifiant la viscosité, les solvants non volatils, des agents perlants, les agents antitranspirants, les dépilatoires, eau parfumée, agent restructurant la peau, les excipients, les charges, les minerais, les anti-irritants, les agents répulsifs d'insectes, les lubrifiants, les pigmentants ou colorants, les agents hypopigmentants, les agents photo-stabilisants, et leurs mélanges, tant qu'ils sont physiquement et chimiquement compatibles avec les autres ingrédients de la composition et spécialement avec les actifs de la présente invention.

Par ailleurs, la nature de ces ingrédients additionnels ne doit pas altérer de manière inacceptable les bénéfices des actifs de l'invention. Ces ingrédients additionnels peuvent être synthétiques ou naturels comme par exemple les extraits de plantes ou venir d'un procédé de biofermentation.

De tels ingrédients additionnels peuvent également être choisis selon leur composition chimique, dans le groupe comprenant : les sucres aminés, glucosamine, D-glucosamine, N-acetyl- glucosamine, N-acetyl-D-glucosamine, mannosamine, N-acetyl mannosamine, galactosamine, N-acétyl galactosamine, vitamine B3 et ses dérivés, le niacinamide, déhydro-acétate de sodium, l'acide déhydroacétique et ses sels, phytosterols, composés d'acide salicylique, hexamidines, composés dihydroxyproline de dialkanoyl, extraits et dérivés de soja, equol, isoflavones, flavonoïdes, phytantriol, farnesol, géraniol, bisabolol, peptides et leurs dérivés, di -, tri -, tétra -, penta -, et hexapeptides et leurs dérivés, lys-thr-thr-lys-ser, palmitoyl-lys-thr-thr-lys-ser, camosine, composés d'acide aminé N-acyl, rétinoïdes, propionate de rétinyl, rétinol, palmitate de rétinyl, acétate de rétinyl, rétinal, acide rétinoïque, vitamines hydrosolubles, ascorbates, vitamine C, glucoside ascorbyl, palmitate ascorbyl, magnésium ascorbyl phosphate, sodium ascorbyl phosphate, vitamines et leurs sels et dérivés, provitamines et leurs sels et dérivés, ethyl panthenol, vitamine B et ses dérivés, vitamine Bl, vitamine B2, vitamine B6, vitamine B12, vitamine K et ses dérivés, acide pantothénique et ses dérivés, éther éthylique de pantothenyl, panthenol et ses dérivés, panthenol ethylique, dexpanthenol, biotine, acides aminés et leurs sels et les dérivés, acides aminés hydrosolubles, asparagine, alanine, indol, acide glutamique, vitamines insolubles dans l'eau, vitamine A, vitamine E, vitamine F , vitamine D et ses composés, mono-, di -, et triterpénoïdes, bêta-ionol, cedrol, et leurs dérivés, acides aminés insolubles dans l'eau, tyrosine, tryptamine, matériaux particulaires, hydroxytoluène butylé, hydroxyanisole butylé, allantoine, nicotinate de tocophérol, tocophérol, esters de tocophérol, palmitoyl-gly-his-lys, phytostérol, hydroxy acides, acide glycolique, acide lactique, acide lactobionique, kétoacides, acide pyruvique, acide phytique, acide lysophosphatidique, stilbenes, cinnamates, resveratrol, kinétine, zeatin, dimethylaminoethanol, peptides naturels, les peptides de soja, sels de sucres acides, gluconate de manganèse, gluconate de zinc, olamine de piroctone, 3,4,4'- trichlorocarbanilide, triclocarban, pyrithione de zinc, hydroquinone, acide kojique, acide ascorbique, magnésium ascorbyl phosphate, ascorbyl glucoside, pyridoxine, l'aloe vera, les alcools de terpène, allantoine, bisabolol, glycyrrhizinate dipotassique, acide de glycérol, de sorbitol, de pentaerythritol, de pyrrolidone et ses sels, dihydroxyacetone, erythrulose, glycéraldéhyde, tartaraldehyde, l'essence de clou de girofle, le menthol, le camphre, l'essence d'eucalyptus, eugénol, le lactate de menthyle, le distillat d'hamamélis, copolymère d'eicosene et vinyl pyrrolidone, iodopropyl butylcarbamate, un polysaccharide, un acide gras essentiel, un salicylate, un acide glycyrrhétinique, des caroténoïdes, des céramides et des pseudo-céramides, un lipide complexe, les huiles en général d'origine naturelle tels le beurre de karité, l'huile d'abricot, l'huile d'onagre, l'huile de pruneau, l'huile de palme, l'huile de monoï, l'huile de kahai, hydroquinone, 1'HEPES, la procystéine, l'O-octanoyl-6-D-maltose, le sel disodique d'acide méthyl glycine diacétique, les stéroïdes tels que la diosgénine et les dérivés de la DHEA, la DHEA déhydroépiandrostérone et/ou un précurseur ou dérivé chimique ou biologique, le N-éthylcarbonyl-4-para-aminophénol, les extraits de myrtille, les phytohormones, les extraits de levure *Saccharomyces cerevisiae*, les extraits d'algues, les extraits de soja, de lupin, de maïs et/ou de pois, l'alvérine et ses sels, en particulier le citrate d'alvérine, les extraits de petits houx et de marron d'inde et leurs combinaisons, un inhibiteur de métalloprotéinases, les extraits de *Schinus molle.*

A titre d'exemples, on peut encore citer :
- les peptides commercialement connus sous le nom MATRIXYL^{®}, ARGIRELINE^{®}, CHRONOGEN^{™}, LAMINIXYL IS^{™}, PEPTIDE Q10^{™}, COLLAXYL ^{™} (brevet FR2827170, ASHLAND^{®}), PEPTIDE VINCI 01^{™} (brevet FR2837098, ASHLAND^{®}), PEPTIDE VINCI 02^{™} (brevet FR2841781, ASHLAND^{®}), ATPeptide^{™} (brevet FR2846883, ASHLAND^{®}) ou encore le peptide de synthèse de séquence Arg-Gly-Ser-NH2, commercialisé sous le nom ATPeptide^{™} par ASHLAND^{®} ;
- l'extrait *d'Artémia salina*, commercialisé sous le nom GP4G^{™} (FR2817748, ASHLAND^{®}) ;
- les extraits peptidiques végétaux tels que les extraits de lin (Lipigénine^{™}, brevet FR2956818, ASHLAND^{®}), les extraits de soja, d'épeautre, de vigne, de colza, de lin, de riz, de maïs, de pois, de cacao ;
- les extraits de levures, par exemple le Dynagen^{™}, (brevet FR2951946, ASHLAND^{®}) ou l'Actopontine^{™} (brevet FR2944526, ASHLAND^{®}) ;

Dans tous les cas, l'homme de métier veillera à ce que ces adjuvants ainsi que leurs proportions soient choisis de telle manière à ne pas nuire aux propriétés avantageuses recherchées de la composition selon l'invention.

Un quatrième objet selon l'invention concerne l'utilisation cosmétique d'une composition selon l'invention, pour améliorer la fonction barrière de la peau, pour améliorer la protection vis-à-vis des toxines, pour améliorer la détoxination de la peau et pour lutter contre l'apparition des signes du vieillissement et diminuer les signes du vieillissement.

La peau est un organe dont la partie supérieure, le *stratum corneum* joue un rôle de barrière physique protectrice vis-à-vis de l'environnement extérieur. Ce rôle de barrière plus communément nommé « fonction barrière de la peau » est d'une importance majeure dans l'homéostasie tissulaire et plus particulièrement dans la protection vis-à-vis d'agents extérieurs ayant un effet néfaste sur la peau, telles que les toxines.

Parmi les toxines, au sens de l'invention, on peut citer les polluants atmosphériques environnementaux, toute substance toxique solide ou solubilisée pouvant entrer en contact avec la peau, ainsi que les toxines produites par les organismes vivants tels que les bactéries ou les champignons.

L'utilisation d'une composition selon l'invention, comprenant une quantité efficace d'un extrait de Condurango, et préférentiellement un extrait de l'écorce de la liane de *Marsdenia cundurango,* permet de mieux protéger la peau vis-à-vis des toxines, en favorisant une meilleure différenciation épidermique et une amélioration de la fonction barrière.

L'utilisation de la composition selon l'invention participe ainsi à la diminution et à la lutte contre l'apparition des signes du vieillissement cutané.

L'autophagie est une voie de dégradation lysosomale, permettant l'élimination des toxines et la dégradation des composants intercellulaires. L'autophagie médiée par une protéine chaperonne (CMA) est l'une des formes sélectives d'autophagie, caractérisée par la présence de LAMP2A (protéine membranaire associée aux lysosomes de type 2A). Les substrats à dégrader (liés à une protéine chaperonne hsc70) se lient à LAMP2 à la surface du lysosome, permettant la translocation dans la lumière lysosomale et son élimination. Le niveau de LAMP2A à la membrane lysosomale diminue avec l'âge, induisant un défaut de l'activité CMA.

Enfin, l'invention a pour cinquième objet une méthode de traitement cosmétique pour augmenter l'expression de récepteurs du goût amer dans les cellules de la peau, comprenant l'application topique d'une composition de l'invention.

La famille des récepteurs couplés aux protéines G nommée TAS2R est présente dans différents organes dont la peau (Reszka E et al. Expression of bitter taste receptors in the human skin in vitro. J Clin Res Bioeth, 2015, 6:218) dans laquelle ils pourraient jouer un rôle dans le maintien de l'homéostasie.

Les récepteurs du goût amer protègent l'organisme contre l'ingestion de substances toxiques, régulent le métabolisme, la satiété, le traitement des aliments et substances ingérées, la détection de l'invasion bactérienne. En particulier, l'activation du récepteur TAS2R38 dans les cellules cutanées par différentes substances, dont des métabolites de plantes et des substances chimiques, conduit à un relargage de calcium dans le cytoplasme et à une dépolarisation de la membrane cellulaire induisant un relargage d'ATP. L'activation de ce récepteur est liée à l'amélioration de la fonction de barrière cutanée (Wölfle U et al. Expression and functional activity of the bitter taste receptors TAS2R1 and TAS2R38 in human keratinocytes. Skin Pharmacol Physiol. 2015;28(3):137-46).

A titre illustratif, des exemples préférés de mode de réalisation de l'invention sont décrits ci-dessous.

L'invention et les avantages qui en découlent seront mieux compris à la lecture de la description et des modes de réalisation non limitatifs qui suivent, rédigés au regard des figures annexées dans lesquelles:
- La figure 1 (exemple 5) présente la quantification de l'expression des récepteurs du goût amer (TAS2R38) dans des biopsies de peau après traitement par un extrait de Condurango à 1 %.
- La figure 2 (exemple 6) présente la quantification de l'expression de l'involucrine dans des biopsies de peau après traitement par un extrait de Condurango à 1 %.
- La figure 3 (exemple 7) présente la quantification de l'expression du marqueur d'autophagie LAMP2 dans des biopsies de peau soumises à un stress multi-toxines puis à un traitement par un extrait de Condurango à 1 %.
- La figure 4 (exemple 8) présente la quantification du relargage de l'interleukine-6 par des biopsies de peau traitées par un extrait de Condurango à 1 % et soumises à un stress par la substance P.

### Exemple 1 : Préparation de la poudre d'écorces de Marsdenia cundurango

Les demandeurs sont en cours de demande d'un permis de Recherche et de Commercialisation auprès du gouvernement Colombien pour avoir accès aux ressources génétiques de *Marsdenia cundurango.*

Les écorces de *Marsdenia cundurango* sont récoltées en Colombie puis séchés au soleil ou par courant d'air chaud dont la température sera comprise entre 40 et 70°C, de façon à obtenir une teneur en humidité résiduelle inférieure à 10%. Les écorces sont broyées dans un broyeur à couteaux comportant une grille de 2 mm, ce qui permet d'obtenir une poudre dont la taille des particules se situe entre 100 et 800 µm, avantageusement entre 300 et 600 µm et plus préférentiellement entre 400 et 500 µm.

### Exemple 2 : Préparation d'un extrait de Condurango par extraction au dioxyde de carbone à l'état supercritique

La poudre obtenue à l'exemple 1 (1,0 kg) est placée dans une cartouche en acier inoxydable, cette cartouche est placée dans un extracteur à fluide supercritique (SFE5, SEPAREX). Le solvant utilisé pour extraire est le dioxyde de carbone à l'état supercritique. Le ratio solvant/matière première est d'environ 40. La pression au sein de l'extracteur est de 230 bar. La température d'extraction est de 50°C.

Le rendement d'extraction est de 0,2% et le produit obtenu est pâteux à température ambiante.

On obtient une teneur en cinnamates de terpényles de l'extrait d'environ 10 à 20% massique.

Cette première forme brute de l'extrait correspond à la définition d'extrait pâteux selon l'invention pouvant être utilisé sous cette forme pour préparer des compositions cosmétiques.

Purification a :
Cette première forme brute de l'extrait est déposée sur une colonne de silice, de taille adaptée à la masse d'extrait à purifier. La séparation des composés d'intérêt est faite par chromatographie flash grâce à un gradient de solvants allant de 100% heptane à un mélange heptane/acétate d'éthyle 9/1 (v/v). La colonne est ensuite rincée en augmentant progressivement la proportion d'acétate d'éthyle dans la phase mobile jusqu'à 100%. Le contenu des différents tubes de récolte est analysé par chromatographie sur couche mince afin de repérer les tubes qui contiennent les composés d'intérêt. Ces derniers sont regroupés puis les solvants sont éliminés à l'évaporateur rotatif afin d'obtenir une fraction contenant 100% de composés cinnamates de terpényles.

La proportion des composés cinnamates de terpényles obtenus est la suivante :
30% de cinnamate de butyrospermyle,
21% de cinnamate de cycloartényle,
23% de cinnamate de β-amyrine,
21% de cinnamate de 24-méthylènecycloartanyle,
5 % de cinnamate de cyclofontumiényle.

La zone d'élution des composés d'intérêt permet d'observer de nombreux pics d'élution. Seuls 5 composés décrits dans la présente invention ont été isolés et étudiés en détails. L'extrait contient donc probablement d'autres composés de la même famille chimique car on observe d'autres pics de polarité similaire à ceux des cinnamates de terpényles décrits. Ces composés ont aussi des spectres UV proches des composés d'intérêt avec notamment un maximum d'absorption proche de 280nm et une fragmentation comparable en spectrométrie de masse.

Purification b :
L'extrait pâteux CO₂ est purifié par distillation moléculaire sur un distillateur à film raclé KDL1 (UIC GmbH), avec un vide poussé de 8 x 10⁻⁴ mbar et une température de 250°C. Le distillat obtenu contient 98% de composés cinnamates de terpényles.

### Exemple 3 : Préparation d'un extrait de Condurango par extraction au dioxyde de carbone supercritique en présence du co-solvant palmitate d'isopropyle

La poudre obtenue à l'exemple 1 (1,0 kg) est placée dans une cartouche en acier inoxydable, cette cartouche est placée dans un extracteur à fluide supercritique (SFE5, SEPAREX). Le solvant utilisé pour extraire est le dioxyde de carbone à l'état supercritique, en présence d'un co-solvant apolaire, le palmitate d'isopropyle. Le ratio solvant/matière première est d'environ 45 et le débit de palmitate d'isopropyle est de 10 ml/minute. La pression au sein de l'extracteur est de 230 bar. La température d'extraction est de 50°C. L'extrait liquide ainsi obtenu peut être séché (déshydraté) sur sulfate de sodium anhydre puis filtré. Après filtration on obtient 1,6 kg d'extrait liquide. Le rendement d'extraction est ainsi proche de 160%.

La teneur en composés cinnamates de terpényles de l'extrait, liquide à température ambiante, est entre 0,5 et 2 % massique.

### Exemple 4 : Préparation d'un extrait de Condurango par extraction au dioxyde de carbone supercritique en présence du co-solvant triglycéride caprylate/caprate de glycérol

La poudre obtenue à l'exemple 1 est placée dans une cartouche en acier inoxydable, cette cartouche est placée dans un extracteur à fluide supercritique. Le solvant utilisé pour extraire est le dioxyde de carbone à l'état supercritique, en présence de triglycéride caprylate/caprate de glycérol, en tant que co-solvant apolaire. Le ratio solvant/matière première est d'environ 45 et le débit de triglycéride caprylate/caprate de glycérol est de 10 ml/minute. La pression au sein de l'extracteur est de 230 bar. La température d'extraction est de 50°C. L'extrait ainsi obtenu peut être séché (déshydraté) sur sulfate de sodium anhydre puis filtré. Après filtration on obtient 1,6 kg d'extrait liquide. Le rendement d'extraction est ainsi proche de 160%.

La teneur en composés cinnamates de terpényles de l'extrait est comprise entre 0,5 et 2 % massique.

### Exemple 5 : Evaluation des effets d'un extrait de Marsdenia cundurango sur l'expression des récepteurs TAS2R38 dans des biopsies de peau :

Le but de cette étude est d'évaluer l'effet d'un traitement par un extrait d'écorce de *Marsdenia cundurango* (Condurango) sur l'expression du récepteur du goût amer TAS2R38 dans des biopsies de peau humaine.

Les récepteurs TAS2R38 sont caractérisés dans cette étude par immunomarquage et témoignent de la capacité de la peau à répondre à différents stimuli de son environnement.

### Protocole:

Des biopsies de peau humaine en culture sont traitées par l'extrait de Condurango obtenu à l'exemple 4 et formulé à 1% (masse/masse) dans une crème appliquée deux fois par jour pendant 48 heures par voie topique (20 µl/biopsie). Des biopsies contrôles reçoivent une crème placebo. Les formules utilisées sont des émulsions classiques huile-dans-eau, réalisées à partir d'ingrédients couramment utilisés. L'amarogentine, molécule amère présente dans les racines de gentiane, est sélectionnée comme activateur des récepteurs TAS2R38 et appliquée en tant que contrôle positif de l'expérience (Extrasynthese, 100 µM). Les biopsies sont maintenues en culture pendant 48 heures.

Puis, la détection des récepteurs TAS2R38 est réalisée par immunomarquage à l'aide d'un anticorps spécifique.

Cette technique est réalisée à partir de coupes en paraffine, incubées en présence de l'anticorps anti-TAS2R38 (polyclonal de lapin, Abcam). Après une nuit d'incubation suivie de rinçages, les coupes sont incubées en présence de l'anticorps secondaire anti-lapin couplé à un fluorophore (Alexa Fluor^{®} 488, Invitrogen). Les coupes sont ensuite examinées au microscope à Epi-fluorescence (Zeiss Axiovert 200M microscope). L'expression de TAS2R38 est alors observée et quantifiée au niveau de l'épiderme.

### Résultats:

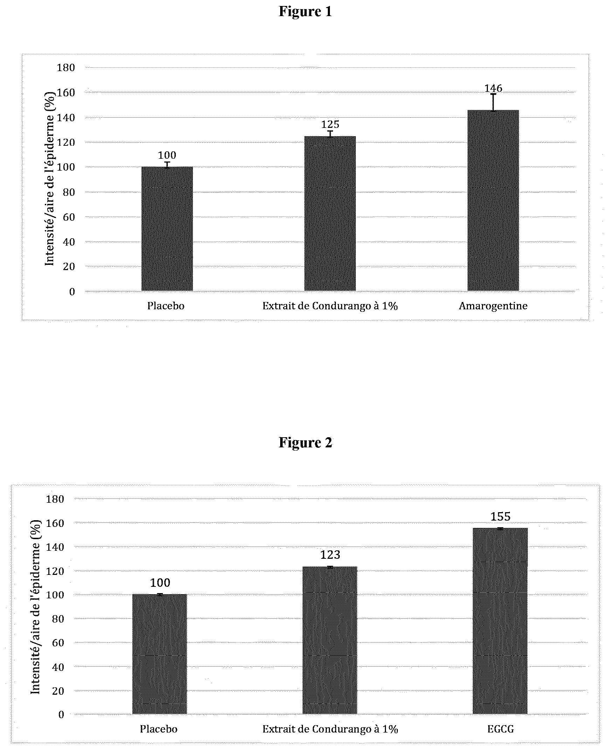

Tel qu'illustré par la figure 1, le traitement avec l'extrait de Condurango à 1% a montré une augmentation de l'intensité de marquage de TAS2R38 dans les biopsies de peau à 48 heures (+25%, très significatif par rapport au placebo).

### Conclusion :

L'application de l'extrait de Condurango a permis d'augmenter l'expression du récepteur du goût amer TAS2R38 dans les biopsies de peau.

### Exemple 6: Evaluation des effets d'un extrait de Condurango sur l'expression de l'involucrine dans des biopsies de peau:

Le but de cette étude est d'évaluer l'effet d'un traitement par l'extrait de Condurango sur l'expression de l'involucrine, en tant que protéine de différenciation, dans l'épiderme humain.

### Protocole:

Des biopsies de peau humaine en culture sont traitées par l'extrait de Condurango obtenu à l'exemple 4 et formulé à 1% (masse/masse) dans une crème, selon le même protocole que dans l'exemple 5. Des biopsies contrôles reçoivent une crème placebo. L'EGCG (Epigallocatechine gallate, Sigma), un polyphénol extrait du thé vert et connu pour augmenter la différenciation des cellules de l'épiderme, est sélectionné comme contrôle positif de cette expérience, dilué à 200 µg/ml. Les biopsies sont maintenues en culture pendant 48 heures.

Puis, la détection de l'involucrine est réalisée par immunomarquage à l'aide d'un anticorps spécifique.

Cette technique est réalisée sur des coupes de peau en paraffine, incubées en présence de l'anticorps anti-involucrine (monoclonal de souris, Novocastra). Après 1 heure d'incubation suivie de rinçages, les coupes sont incubées en présence de l'anticorps secondaire anti-souris couplé à un fluorophore (Alexa Fluor^{®} 488, Invitrogen). Les coupes sont ensuite examinées au microscope à Epi-fluorescence (Zeiss Axiovert 200M microscope). L'expression d'involucrine est alors observée et quantifiée au niveau de l'épiderme.

### Résultats:

Tel qu'illustré par la figure 2, l'extrait de Condurango à 1% a entraîné une augmentation de l'intensité du marquage de l'involucrine, dans les biopsies traitées pendant 48 heures (+23%, hautement significatif).

### Conclusion :

Ce test *ex vivo* permet de conclure à un effet positif de l'extrait de Condurango sur l'expression de l'involucrine, en liaison avec la fonction barrière cutanée.

### Exemple 7 : Evaluation des effets de l'extrait de Condurango sur l'expression du marqueur d'autophagie LAMP2 après un stress multi-toxines dans des biopsies de peau en culture

Le but de cette étude est d'observer l'effet du traitement par Condurango sur des biopsies de peau préalablement soumises à un stress multi-toxines, réalisé en incubant les biopsies avec de la fumée de cigarette.

Le marqueur choisi est la protéine membranaire lysosomale LAMP2A qui intervient dans les processus d'autophagie, une des principales voies de dégradation des déchets cellulaires.

### Protocole :

Des biopsies de peau humaine en culture sont placées dans une chambre étanche saturée en fumée de cigarette pendant 30 minutes. Ce stress est désigné par stress multi-toxique, en raison de la composition de la fumée de cigarette en différentes classes de molécules toxiques. Après le stress, les biopsies sont mises en culture et traitées par l'extrait de Condurango obtenu à l'exemple 4 et formulé à 1% (masse/masse) dans une crème, comme dans l'exemple 5. Des biopsies contrôles sans stress sont incubées dans les mêmes conditions. L'EGCG (Epigallocatechine gallate, Sigma), polyphénol de thé vert, est utilisé en tant que contrôle positif de cette expérience, dilué à 200 µg/ml.

Au terme de cette incubation, la protéine LAMP2 est détectée par immunomarquage sur des coupes de biopsies de peau.

Cette technique est réalisée sur des coupes de peau en paraffine, incubées en présence de l'anticorps anti-LAMP2 (polyclonal de lapin, Abcam). Après 1 heure d'incubation suivie de rinçages, les coupes sont incubées en présence de l'anticorps secondaire anti-lapin couplé à un fluorophore (Alexa Fluor^{®} 488, Invitrogen). Les coupes sont ensuite examinées au microscope à Epi-fluorescence (Zeiss Axiovert 200M microscope). L'expression de LAMP2 est alors observée et quantifiée au niveau de l'épiderme.

### Résultats :

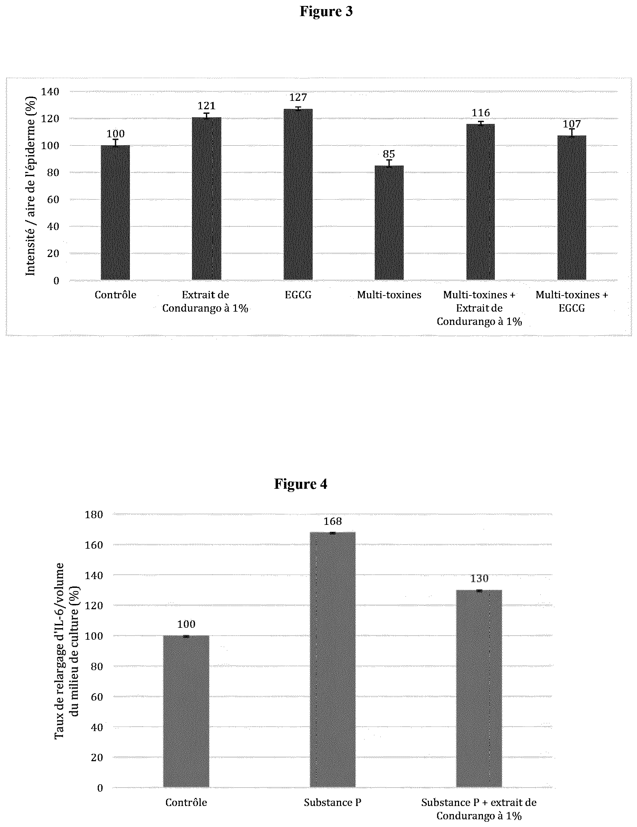

Tel qu'illustré par la figure 3, en l'absence de stress, le traitement par l'extrait de Condurango à 1% entraîne une augmentation de l'intensité de marquage de LAMP2 dans les biopsies de peau à 48 heures (+21%, très significatif), proche de celle obtenue avec le contrôle positif EGCG (+27%, hautement significatif). Le stress multi-toxique induit une diminution de LAMP2 (-15%, significatif), alors qu'en présence de l'extrait de *Condurango,* une augmentation est observée, compensant l'effet du stress (+36%, hautement significatif, contre +26%, significatif pour le contrôle EGCG).

### Conclusion :

En absence de stress, l'extrait de Condurango est favorable à la protéostasie en augmentant l'expression du marqueur d'autophagie LAMP2. En présence d'un stress multi-toxique, l'extrait de Condurango est favorable à l'élimination des dommages induits par le stress dans les biopsies de peau.

### Exemple 8 : Effet de l'extrait de Condurango sur le relargage de l'interleukine-6 par des biopsies de peau en culture soumises à un stress par la substance P

Le but de cette expérience est d'étudier l'effet de l'extrait de Condurango sur des biopsies de peau soumises à un stress par la substance P. La substance P est le médiateur central de l'inflammation neurogénique (inflammation médiée par le système nerveux périphérique). Ce neuropeptide est impliqué dans différents processus incluant l'inflammation aigüe ou chronique, et la perception de la douleur ou nociception. L'effet du stress réalisé ou non en présence de l'extrait de Condurango est étudié en mesurant le relargage de l'interleukine-6 (IL-6), une cytokine synthétisée par les cellules cutanées et considérée comme un médiateur de l'inflammation nociceptive (O'Donovan A et al. Clinical anxiety, cortisol and interleukin-6: evidence for specificity in emotion-biology relationships. Brain Behav Immun. 2010 Oct;24(7): 1074-7).

### Protocole :

Des biopsies de peau en culture sont traitées par l'extrait de Condurango obtenu à l'exemple 4, à 1% formulé dans une crème, comme dans l'exemple 5 avec ou sans ajout de substance P à 10⁻⁶ M dans le milieu de culture.

Après 48 heures d'incubation, le milieu de culture des biopsies est collecté et le taux d'IL-6 est mesuré par ELISA.

### Résultats :

Tel qu'illustré dans la figure 4, le stress à la substance P a entraîné une augmentation du taux d'IL-6 de +68% par rapport au contrôle non stressé. Le traitement par l'extrait de Condurango à 1% a réduit le taux d'IL-6 (+30% par rapport au contrôle non stressé).

### Conclusion :

L'extrait de Condurango a permis de limiter le stress neurogénique induit par la substance P dans les biopsies de peau.

### Exemple 9 : Formulation d'une Emulsion marine hydratante

| Ingrédient / Nom commercial | Nom INCI | | % |
|---|---|---|---|
| Phase A | | | |
| Eau purifiée | Water/Aqua | | Qs. 100 |
| Disodium EDTA | Disodium EDTA | | 0,10 |
| Stabileze^{™} QM polymer | PVM/MA Decadiene Crosspolymer | | 0,50 |
| Lubrajel^{™} Marine^{∗} hydrogel | Water/Aqua (and) Glycerin (and) Sodium PCA (and) Erythritol (and) Carrageenan (and) Xanthan Gum | | 4,00 |
| Phase B | | | |
| ProLipid^{™} 141 lamellar Gel | Glyceryl Stearate (and) Behenyl Alcohol (and) Palmitic Acid (and) Stearic Acid (and) Lecithin (and) Lauryl Alcohol (and) Myristyl Alcohol (and) Cetyl Alcohol | | 4,00 |
| Refined Shea Butter | Butyrospermum Parkii (Shea) Butter | | 1,00 |
| Emulsynt^{™} GDL ester | Glyceryl Dilaurate | | 2,00 |
| Ceraphyl^{™} ODS ester | Octyldodecyl Stearate | | 2,00 |
| Ceraphyl SLK ester | Isodecyl Neopentanoate | | 5,00 |
| Ceraphyl 368 ester | Ethylhexyl Palmitate | | 3,00 |
| Belsil^{™∗} PDM 20 | Trimethylsiloxyphenyl Dimethicone | | 3,00 |
| Extrait de Condurango selon exemple 4 | Marsdenia Condurango Bark Extract | | 1,00 |
| Phase C | | | |
| Sodium Hydroxide | Sodium Hydroxide | | 0,13 |
| Eau purifiée | Water/Aqua | | 2,00 |
| Phase D | | | |
| Optiphen^{™} DP preservative | Propylene carbonate (and) Benzoic Acid (and) Dehydroacetic Acid (and) Propanediol | | 1,00 |
| Optiphen OD preservative booster | Caprylyl Glycol | | 0,50 |
| Phase E | | | |
| Eau purifiée | Water/Aqua | | 10,00 |
| Natrosol^{™} Plus 330 CS HMHEC | Cetyl Hydroxyethylcellulose | | 0,10 |
| Phase F | | | |
| PF Mineral Defense 8509133 | Fragrance/Parfum (and) Butylphenyl methylpropional (Lilial) (and) Hydroxycitronellal | | 0,20 |

Méthode de préparation:
1. Dans le récipient principal, commencer à chauffer à 75°C et ajouter les ingrédients de la phase A, un par un tout en mélangeant jusqu'à homogénéisation complète.
2. Dans un 2ème bécher, préparer la phase B et chauffer à 75°C.
3. A 75°C, verser la phase B dans la phase A et mélanger jusqu'à homogénéisation complète.
4. Prémélanger la phase C et ajouter dans le récipient principal à 60°C
5. A 45°C, ajouter les ingrédients de la phase D, un par un, tout en mélangeant jusqu'à homogénéisation complète après chaque ingrédient.
6. Dans un bécher à part préparer la phase E : saupoudrer le Natrosol dans l'eau à température ambiante et homogénéiser tout en chauffant à 60°C.
7. A 30°C, ajouter la phase E et mélanger bien.
8. A à température ambiante, ajouter la phase F et mélanger bien.
9. Arrêter à 25°C.
10. Apparence: Emulsion blanche ; pH: 4,8 - 5,3 ; Viscosité (D0): 25000 - 35000 cps (Brookfield RVT/Spindle B/5 RPM/1 minute/25°C)
11. La conservation de la formule a été validée par un double test d'efficacité après 28 jours. Toutefois, les conservateurs n'ont pas été optimisés à leur plus bas niveau d'efficacité.

## Revendications

1. Procédé d'obtention d'un extrait de *Marsdenia cundurango* comprenant des composés cinnamates de terpényles, **caractérisé en ce qu'**il comprend les étapes suivantes :
a) on recueille l'écorce et/ou le bois des lianes de *Marsdenia cundurango* qui sont séchés puis broyés;
b) on réalise une extraction avec du dioxyde de carbone à l'état supercritique, à une température comprise entre 45 et 55°C et à une pression comprise entre 200 et 260 bar au sein de l'extracteur,
c) on récupère l'extrait de *Marsdenia cundurango,*
d) éventuellement on purifie l'extrait obtenu à l'étape c).

2. Procédé selon la revendication 1, **caractérisé en ce que** l'extrait de *Marsdenia cundurango* contient au moins 10 % en poids de composés cinnamates de terpényles.

3. Procédé selon la revendication 1 **caractérisé en ce qu'**à l'étape b) on utilise en outre au moins un co-solvant apolaire.

4. Procédé selon la revendication 3 **caractérisé en ce que** le co-solvant apolaire est un solvant agro-sourcé choisi parmi des huiles végétales, des esters d'acides et d'alcools à chaîne plus ou moins longue tels que par exemple l'acétate d'éthyle, le lactate d'éthyle, le propionate d'éthyle, le palmitate d'isopropyle, l'oléate d'éthyle, le stéarate de méthyle, l'oléate d'oleyle, le triéthyl citrate, le triglycéride caprylate/caprate de glycérol ou encore des hydrocarbures agro-sourcés en C10 à C14, ou une combinaison de ces solvants.

5. Extrait de l'écorce et/ou du bois de la liane de *Marsdenia cundurango* obtenu par le procédé selon l'une des revendications 1 ou 2, **caractérisé en ce qu'**il est pâteux et comprend des composés cinnamates de terpényles en une concentration d'au moins 10% en poids du poids total de l'extrait.

6. Extrait de l'écorce et/ou du bois de la liane de *Marsdenia cundurango* obtenu par le procédé selon l'une des revendications 3 ou 4, **caractérisé en ce que** l'extrait est sous forme liquide et comprend des composés cinnamates de terpényles en une concentration de 0,5% à 2% en poids du poids total de l'extrait.

7. Extrait de *Marsdenia cundurango* selon l'une des revendications 5 ou 6, **caractérisé en ce que** les composés cinnamates de terpényles sont de formule générale suivante I : 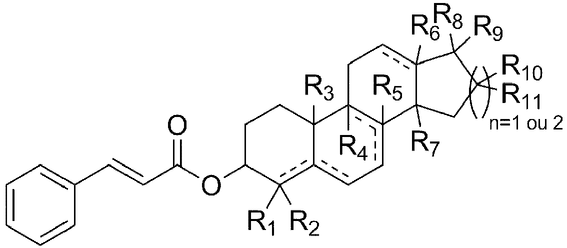 dans laquelle :
- les radicaux R1, R2, R3, R4, R5, R6 et R7 représentent des protons, des méthyles ou des chaînes alkyles oxydées ou non oxydées, saturées ou non saturées,
- les radicaux R8, R9, R10 et R11 représentent des protons, des méthyles, des groupements alkyles cycliques ou non cycliques, saturés ou non saturés, oxydés ou non oxydés,
- les pointillés illustrent la présence potentielle d'une liaison insaturée à cette position.

8. Extrait selon la revendication 7, **caractérisé en ce que** les composés cinnamates de terpényles sont notamment le cinnamate de β-amyrine, le cinnamate de 24-méthylènecycloartanyle, le cinnamate de butyrospermyle, le cinnamate de cycloartényle et le cinnamate de cyclofontumiényle.

9. Extrait selon l'une quelconque des revendications 5 à 8 **caractérisé en ce que** l'extrait provient de l'écorce de la liane de *Marsdenia cundurango.*

10. Composition cosmétique, **caractérisée en ce qu'**elle comprend, à titre d'agent actif, une quantité efficace d'un extrait de *Marsdenia cundurango* selon l'une quelconque des revendications 6 à 9 et un excipient physiologiquement acceptable.

11. Composition selon la revendication 10, **caractérisée en ce que** l'extrait de *Marsdenia cundurango* est présent dans la composition à une concentration de 0,001 à 10% en poids par rapport au poids total de la composition, préférentiellement de 0,01 à 5%.

12. Composition selon l'une des revendications 10 ou 11, **caractérisée en ce qu'**elle est formulée pour être appliquée topiquement sur la peau.

13. Utilisation cosmétique d'une composition selon l'une des revendications 10 à 12 pour améliorer la fonction barrière de la peau, pour améliorer la protection vis-à-vis des toxines, pour augmenter les capacités de détoxination de la peau et diminuer les signes du vieillissement.

14. Méthode de traitement cosmétique pour augmenter l'expression de récepteurs du goût amer dans les cellules de la peau, comprenant l'application topique d'une composition selon l'une des revendications 10 à 12.

## Patentansprüche

1. Verfahren zum Erhalten eines Extrakts von *Marsdenia cundurango,* umfassend Terpenylcinnamat-Verbindungen, **dadurch gekennzeichnet, dass** es die folgenden Schritte umfasst:
a) Sammeln der Rinde und/oder des Holzes der Lianen von *Marsdenia cundurango,* die getrocknet und dann zerkleinert werden;
b) Durchführen einer Extraktion mit Kohlendioxid im superkritischen Zustand, bei einer Temperatur zwischen 45 und 55 °C und bei einem Druck zwischen 200 und 260 bar im Inneren des Extraktors,
c) Sammeln des Extrakts von *Marsdenia cundurango,*
d) gegebenenfalls Reinigen des Extrakts, der in dem Schritt c) erhalten wurde.

2. Verfahren nach Anspruch 1, **dadurch gekennzeichnet, dass** der Extrakt von *Marsdenia cundurango* mindestens 10 Gew.-% Terpenylcinnamat-Verbindungen enthält.

3. Verfahren nach Anspruch 1, **dadurch gekennzeichnet, dass** in dem Schritt b) außerdem mindestens ein apolares Co-Lösungsmittel verwendet wird.

4. Verfahren nach Anspruch 3, **dadurch gekennzeichnet, dass** das apolare Co-Lösungsmittel ein Lösungsmittel agrarischen Ursprungs ist, ausgewählt aus Pflanzenölen, Säure- und Alkoholestern mit mehr oder weniger langer Kette, wie zum Beispiel Ethylacetat, Ethyllactat, Ethylpropionalt, Isopropylpalmitat, Ethyloleat, Methylstearat, Oleyloleat, Triethylcitrat, dem Triglycerid Caprylat/Capratglycerin, oder auch C10- bis C14-Kohlenwasserstoffen agrarischen Ursprungs, oder einer Kombination dieser Lösungsmittel.

5. Rinden- und/oder Holzextrakt der Liane von *Marsdenia cundurango,* welcher durch das Verfahren nach einem der Ansprüche 1 oder 2 erhalten wird, **dadurch gekennzeichnet, dass** er pastenförmig ist und Terpenylcinnamat-Verbindungen in einer Konzentration von mindestens 10 Gew.-%, bezogen auf das Gesamtgewicht des Extrakts, umfasst.

6. Rinden- und/oder Holzextrakt der Liane von *Marsdenia cundurango,* welcher durch das Verfahren nach einem der Ansprüche 3 oder 4 erhalten wird, **dadurch gekennzeichnet, dass** der Extrakt in flüssiger Form ist und Terpenylcinnamat-Verbindungen in einer Konzentration von mindestens 0,5 Gew.-% bis 2 Gew.-%, bezogen auf das Gesamtgewicht des Extrakts, umfasst.

7. Extrakt von *Marsdenia cundurango* nach einem der Ansprüche 5 oder 6, **dadurch gekennzeichnet, dass** die Terpenylcinnamat-Verbindungen die folgende allgemeine Formel I aufweisen: 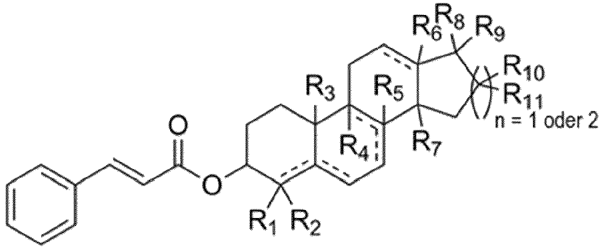 wobei:
- die Reste R1, R2, R3, R4, R5, R6 und R7 Protonen, Methyle oder oxidierte oder nicht oxidierte, gesättigte oder ungesättigte Alkylketten darstellen,
- die Reste R8, R9, R10 und R11 Protonen, Methyle, cyclische oder nicht cyclische, gesättigte oder ungesättigte, oxidierte oder nicht oxidierte Alkylketten darstellen,
- die durchbrochenen Linien die potentielle Anwesenheit einer ungesättigten Bindung in dieser Position veranschaulichen.

8. Extrakt nach Anspruch 7, **dadurch gekennzeichnet, dass** die Terpenylcinnamat-Verbindungen insbesondere β-Amyrincinnamat, 24-Methylencycloartanylcinnamat, Butyrospermylcinnamat, Cycloartenylcinnamat und Cyclofontumienylcinnamat sind.

9. Extrakt nach einem der Ansprüche 5 bis 8 **dadurch gekennzeichnet, dass** der Extrakt von der Rinde der Liane von *Marsdenia cundurango* stammt.

10. Kosmetische Zusammensetzung, **dadurch gekennzeichnet, dass** sie als aktiven Bestandteil eine wirksame Menge eines Extrakts von *Marsdenia cundurango* nach einem der Ansprüche 6 bis 9 und einen physiologisch annehmbaren Exzipienten umfasst.

11. Zusammensetzung nach Anspruch 10, **dadurch gekennzeichnet, dass** der Extrakt von *Marsdenia cundurango* in der Zusammensetzung in einer Konzentration von 0,001 bis 10 Gew.-%, bezogen auf das Gesamtgewicht der Zusammensetzung, vorzugsweise von 0,01 bis 5 %, vorhanden ist.

12. Zusammensetzung nach einem der Ansprüche 10 oder 11, **dadurch gekennzeichnet, dass** sie formuliert ist, um topisch auf die Haut aufgetragen zu werden.

13. Kosmetische Verwendung einer Zusammensetzung nach einem der Ansprüche 10 bis 12, zur Verbesserung der Barrierefunktion der Haut, zur Verbesserung des Schutzes gegen Toxine, zur Erhöhung der Detoxifizierungskapazität der Haut und zur Verringerung der Anzeichen der Alterung.

14. Verfahren zur kosmetischen Behandlung, um die Expression von Bitterstoffrezeptoren in den Hautzellen zu erhöhen, umfassend das topische Auftragen einer Zusammensetzung nach einem der Ansprüche 10 bis 12.

## Claims

1. A method for obtaining a *Marsdenia cundurango* extract comprising terpinyl cinnamate compounds, **characterised in that** the method comprises the following steps:
a) the bark and/or wood of *Marsdenia cundurango* creepers are/is collected, dried and then milled;
b) an extraction is performed with carbon dioxide in the supercritical state at a temperature between 45 and 55°C and at a pressure between 200 and 260 bar within the extractor,
c) the *Marsdenia cundurango* extract is recovered,
d) the extract obtained in step c) is optionally purified.

2. The method according to claim 1, **characterised in that** the *Marsdenia cundurango* extract contains at least 10% by weight of terpinyl cinnamate compounds.

3. The method according to claim 1, **characterised in that** in step b) at least one apolar co-solvent is also used.

4. The method according to claim 3, **characterised in that** the apolar co-solvent is an agro-sourced solvent selected from vegetable oils, acid esters and long- or short-chain alcohols, such as ethyl acetate, ethyl lactate, ethyl propionate, isopropyl palmitate, ethyl oleate, methyl stearate, oleyl oleate, triethyl citrate, glycerol caprylate/caprate triglyceride, or agro-sourced C10 to C14 hydrocarbons, or a combination of these solvents.

5. A *Marsdenia cundurango* creeper bark and/or wood extract obtained by the method according to one of claims 1 or 2, **characterised in that** it is pasty and comprises terpinyl cinnamate compounds in a concentration of at least 10% by weight of the total weight of the extract.

6. A *Marsdenia cundurango* creeper bark and/or wood extract obtained by the method according to one of claims 3 or 4, **characterised in that** the extract is in liquid form and comprises terpinyl cinnamate compounds in a concentration of from 0.5% to 2% by weight of the total weight of the extract.

7. The *Marsdenia cundurango* extract according to one of claims 5 or 6, **characterised in that** the terpinyl cinnamate compounds are of the following general formula I: 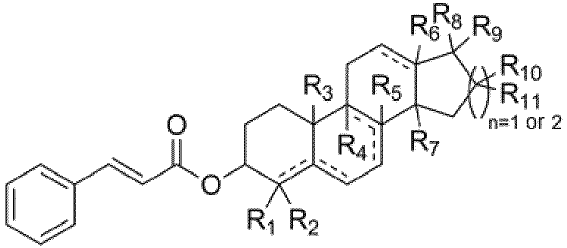 in which:
- the radicals R1, R2, R3, R4, R5, R6 and R7 represent protons, methyls or saturated or non-saturated oxidised or non-oxidised alkyl chains,
- the radicals R8, R9, R10 and R11 represent protons, methyls, or oxidised or non-oxidised, saturated or non-saturated, cyclic or non-cyclic alkyl groups,
- the dotted lines illustrate the potential presence of an unsaturated bond at this position.

8. The extract according to claim 7, **characterised in that** the terpinyl cinnamate compounds are in particular β-amyrin cinnamate, 24-methylene cycloartanyl cinnamate, butyrospermyl cinnamate, cycloartenyl cinnamate and cyclofontumienyl cinnamate.

9. The extract according to any one of claims 5 to 8, **characterised in that** the extract originates from *Marsdenia cundurango* creeper bark.

10. A cosmetic composition, **characterised in that** it comprises, by way of active agent, an effective amount of a *Marsdenia cundurango* extract according to any one of claims 6 to 9 and a physiologically acceptable excipient.

11. The composition according to claim 10, **characterised in that** the *Marsdenia cundurango* extract is present in the composition at a concentration of from 0.001 to 10% by weight in relation to the total weight of the composition, preferably from 0.01 to 5%.

12. The composition according to one of claims 10 or 11, **characterised in that** it is formulated for topical application to the skin.

13. Cosmetic use of a composition according to one of claims 10 to 12 for improving the barrier function of the skin, for improving protection against toxins, for increasing the detoxification capabilities of the skin, and for reducing the signs of ageing.

14. A cosmetic treatment method for increasing the expression of bitter taste receptors in the skin cells, comprising the topical application of a composition according to one of claims 10 to 12.
